# NEUE EUROPÄISCHE PATENTSCHRIFT

(11) **EP 0 929 234 B2**
(45) Veröffentlichungstag und Bekanntmachung des Hinweises auf die Entscheidung über den Einspruch: **05.10.2005**
(45) Hinweis auf die Patenterteilung: 04.12.2002
(21) Anmeldenummer: 97918971.9
(22) Anmeldetag: 09.08.1997
(51) Int. Cl.: A23L 1/236, A61K 9/20, A61K 47/26

(54) **KOMPRIMATE, ENTHALTEND EIN SÜSSUNGSMITTELGEMISCH**
TABLETS CONTAINING A SWEETENING MIXTURE
COMPRIMES CONTENANT UN MELANGE EDULCORANT

(30) Priorität: 25.09.1996 DE 19639343
(43) Veröffentlichungstag der Anmeldung: 21.07.1999
(73) Patentinhaber: Südzucker Aktiengesellschaft Mannheim/Ochsenfurt, 68165 Mannheim (DE)
(72) Erfinder: RAPP, Knut, M., D-67591 Offstein (DE); WILLIBALD-ETTLE, Ingrid, D-76829 Landau (DE)
(74) Vertreter: Schrell, Andreas
(86) Internationale Anmeldenummer: PCT/EP1997/004346
(87) Internationale Veröffentlichungsnummer: WO 1998/012936

(56) Entgegenhaltungen:
- EP-A- 0 028 905
- EP-A- 0 625 578
- WO-A-97/30598
- DÖRR TILLMANN , WILLIBALD-ETTLE INGRID: "BEURTEILUNG DER AUFLÖSEKINETIK ORALEN PHARMAZEUTISCHER DARREICHUNGSFORMEN AUS VERSCHIEDENEN SACCHARIDEN UND ZUCKERAUSTAUSCHSTOFFEN" DIE PHARMAZEUTISCHE INDUSTRIE, Bd. 58, Nr. 10, 1996, Seiten 947-952, XP002050750
- LICHTENTHALTER FRIEDER W. , LINDNER HANS J.: "THE PREFERRED CONFORMATIONS OF GLYCOSYLALDITOLS" LIEBIG'S ANNALEN DER CHEMIE, 1981, Seiten 2372-2383, XP002050751
- Carbohydrate Research 164 (1987), 477-485
- Die Ernährungsindustrie 11/02, pages 28-33
- Nationales ZDS-Praktikum, 11-15 December 1995

## Beschreibung

Die vorliegende Erfindung betrifft Komprimate, die ein Süßungsmittelgemisch aus 6-O-α-D-Glucopyranosyl-D-sorbit, 1-O-α-D-Glucopyranosyl-D-sorbit und 1-O-α-D-Glucopyranosyl-D-mannit enthalten sowie die Verwendung dieser Süßungsmittelgemische in Komprimaten.

Komprimate sind aus zusammengepreßten Bestandteilen bestehende Genuß-, Arznei- oderauch Nahrungsmittel. Komprimate enthalten dementsprechend im allgemeinen ein Träger- oder Verdünnungsmittel, Bindemittel, Trenn- oder Gleitmittel sowie die aktiven Wirkstoffe wie Geschmacksstoffe, Arzneistoffe oder Süßungsmittel. Als Träger- beziehungsweise Verdünnungsmittel wird häufig Saccharose, Lactose, Glucose, Stärke oder Mannit verwendet. Die Verwendung dieser Träger- beziehungsweise Verdünnungsmittel weist den Nachteil auf, daß zusätzlich Bindemittel benötigt werden, um eine ausreichende Komprimierbarkeit zu gewährleisten.

Bayerhöhler in "die Ernährungsindustrie 11 (1992) 28 ff offenbart komprimate, die Isomalt enthalten und deren Härte durch Zustaz anderer Zuckeraustauschstoffe erhöht werden kann.

Fritzsching in "Nationales ZDS-Praktikum PNZ-45, Zuckerfrei Dragees, 11-15. 12. 1995 " offenbart komprimate, die Isomalt und zur Erhöhung der Tablettenhärte einen Zusatz von z.B Sorbit enthalten.

Die EP-B1 0 0 28 905 beschreibt die Verwendung von Isomaltulose als Verdünnungsmittel in Tabletten. Isomaltulose weist jedoch nur eine vergleichsweise geringe Süße auf.

Die EP-A-0 625 578 offenbart ein Süßungsmittel aus 6-O-α-D-Glucopyranosyl-D-sorbit, 1-O-α-D-Glucopyranosyl-D-sorbit und 6-O-α-D-Glucopyranosyl-D-mannit sowie Erdbeerkonfitüre, Karamellen und Speiseeis, die dieses Süßungsmittel enthalten. Dörr und Willibald-Ettle in Pharm. Ind. 58 (10), 1996, 947 bis 952 offenbaren Komprimate, die Sorbit, Xylit, Lactit oder Isomalt^{R} enthalten. Die dort beschriebenen Komprimate zeichnen sich durch ein bestimmtes Löslichkeitsverhalten sowie verbesserungsfähige Komprimierbarkeit aus. Lichtenthaler und Lindner in Liebigs Ann. Chem., 1981, 2372 bis 2383, offenbaren Analysen zur Kristallstruktur von Isomaltit.

Das der Erfindung zugrunde liegende technische Problem besteht also darin, Komprimate bereitzustellen, die die vorgenannten Nachteile überwinden, insbesondere eine verbesserte Süße, Löslichkeit und Komprimierbarkeit aufweisen.

Die Lösung dieses technischen Problems liegt in der Bereitstellung der im Anspruch 1 gekennzeichneten Komprimate. Die erfindungsgemäßen Komprimate weisen aufgrund ihres Gehaltes an 1,1-GPS, insbesondere aufgrund ihres Gehaltes an Süßungsmittelgemisch aus 1,6-GPS, 1,1-GPS und 1,1-GPM eine gegenüber handelsübliches Isomalt^{R} (äquimolares Gemisch aus 1,6-GPS und 1,1-GPM, hydrierte Isomaltulose) enthaltenden Komprimaten verbesserte Löslichkeit und Süßkraft auf. Die erfindungsgemäßen Komprimate weisen den überraschenden Vorteil auf, daß sie sich ohne Verwendung von Bindemitteln herstellen lassen und eine verbesserte Komprimierbarkeit aufweisen, das heißt, zum Erhalt einer bestimmten Härte ist ein vergleichsweise geringerer Preßdruck nötig. Weitere mit der verbesserten Komprimierbarkeit einhergehende Vorteile der erfindungsgemäßen Komprimate liegen in ihrer großen Härte, die mit verhältnismäßig geringem Hauptpreßdruck erzielt wird.

Weitere vorteilhafte Ausgestaltungen der Erfindung sind den abhängigen Ansprüchen entnehmbar.

Die Erfindung betrifft Komprimate, die ein Süßungsmittelgemisch aus 10 bis 50 Gew.-% 1,6-GPS, 2 bis 20 Gew.-% 1,1-GPS und 30 bis 70 Gew.-% 1,1-GPM, bezogen auf das Gewicht des Süßungsmittelgemisches, enthalten.

Gemäß der Erfindung weisen die Komprimate 50 bis 99 Gew.-%, bezogen auf das Gewicht des Komprimates, 1,1-GPS beziehungsweise Süßungsmittelgemisch auf. Die Komprimate können zusätzlich Monosaccharide, Disaccharide, Monosaccharidalkohole, Disaccharidalkohole, Stärke, Stärkederivate, Cellulose, Cellulosederivate oder Inulin enthalten. Die Komprimate können auch insbesondere Sorbit, Mannit, hydrierte oder nicht-hydrierte Oligosaccharide, Xylit oder Zucker, wie Saccharose, Glucose, Fructose oder Xylose enthalten. Diese liegen jedoch vorteilhafterweise in einer Menge von weniger als 30 Gew.-%, vorzugsweise weniger als 5 Gew.-%, bezogen auf das Gewicht des Komprimates, vor. In besonders vorteilhafter Ausführung sind die erfindungsgemäßen Komprimate jedoch zuckerfrei und damit brennwert-reduziert und diabetiker-geeignet.

In einer besonders bevorzugten Ausführungsform der Erfindung ist vorgesehen, daß die Komprimate zusätzlich Intensiv-Süßstoffe wie Acesulfam-K, Aspartam, Cyclamat, Glycyrrhizin, Thaumatin, Saccharin oder ähnliche enthalten. In vorteilhafter Weise enthalten die erfindungsgemäßen Komprimate zudem Geschmacks- oder Aromastoffe wie Zitronen- oder Pfefferminz-Aroma. Die erfindungsgemäßen Komprimate können auch lebensmittelverträgliche Säuren wie Ascorbinsäure oder Zitronensäure sowie als Gleitmittel Fettsäuren oder deren Salze wie Magnesiumsterat oder Natriumstearat enthalten. Schließlich kann vorgesehen sein, daß in den erfindungsgemäßen Komprimaten Farbstoffe und/oder Sprengmittel, wie Bicarbonat oder Carboxylmethylcellulose enthalten sind.

In einer besonders bevorzugten Ausführungsform ist vorgesehen, Komprimate bereitzustellen, die pharmazeutisch aktive Wirkstoffe in den Mund- und Rachenraum bringen und dort freisetzen können. Im Zusammenhang der vorliegenden Erfindung sind unter pharmazeutisch aktiven Wirkstoffen Substanzen zu verstehen, die einen erwünschten prophylaktischen oder therapeutischen Effekt auf den menschlichen oder tierischen Körper haben. Diese Substanzen dienen_ also insbesondere der Prophylaxe oder Therapie von Mangelzuständen oder Krankheitsbildern. Erfindungsgemäß können beispielsweise Enzyme, Coenzyme, Mineralstoffe, Vitamine, Antibiotika, mikrobizid oderfungizid wirkende Stoffe, Nikotin, Coffein, Eukalyptus, Codein, Phenacetin, Acetylsalicylsäure, Menthol oder andere pharmazeutisch aktive Wirkstoffe in die Komprimate eingeschlossen werden. Die pharmazeutisch aktiven Wirkstoffe sind in einer Menge vorzusehen, daß sie den erwünschten pharmazeutischen Effekt bewirken. Die schonende Verarbeitung der Komprimate sowie deren besonderes Löslichkeitsverhalten machen die erfindungsgemäßen Komprimate besonders geeignet, pharmazeutisch aktive Wirkstoffe in den Mund- und Rachenraum zu verbringen. Isomalt^{R} enthaltende sowie zuckerhaltige Komprimate lösen sich vergleichsweise schlechter, so daß die Wirkstoff-Freigabe nur mit Verzögerung erfolgt. Die Wirkstoff-Freigabe erfindungsgemäßer Komprimate setzt in vorteilhafter Weise rasch und lang andauemd ein.

In einer weiteren Ausführungsform betrifft die Erfindung Komprimate in Form von Lutsch- oder Kautabletten.

Schließlich offenbart die Erfindung die Verwendung von 1,1-GPS beziehungsweise das Süßungsmittelgemisches aus 1,6-GPS, 1,1-GPM und 1,1-GPS in einem Pulvergemisch zur Herstellung eines Komprimats zur Verbesserung von dessen Komprimierbarkeit.

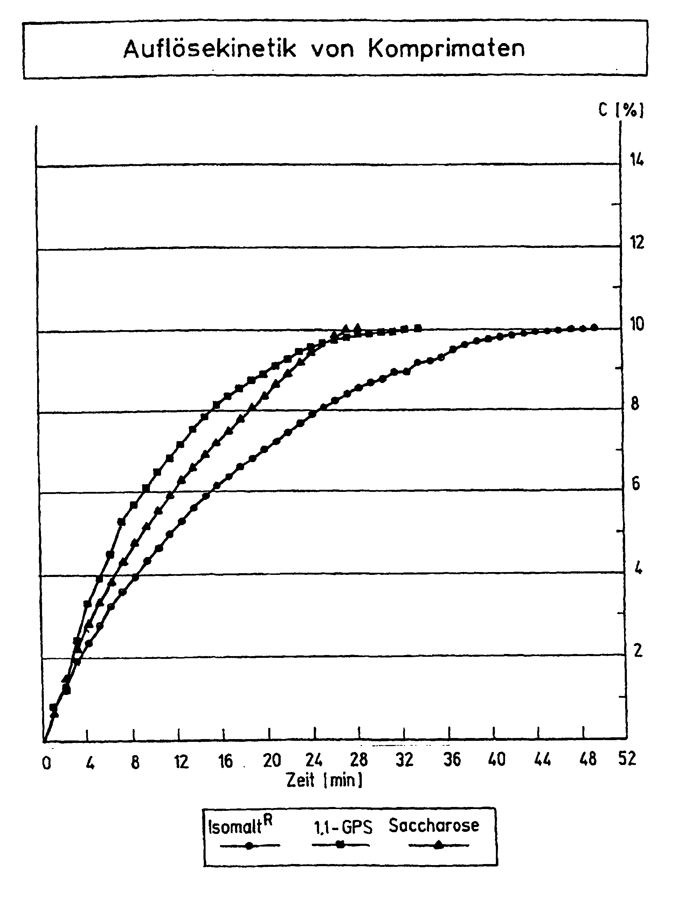

Die folgenden Beispiele und die Figur erläutern die Erfindung in Einzelheiten.

Die Figur stellt in graphischer Form Auflösekinetiken erfindungsgemäßer und konventioneller Komprimate dar.

### Beispiel 1: Herstellung von Lutschtabletten (Kautabletten)

### Rezeptur

| | |
|---|---|
| Süßungsmittelgemisch, enthaltend 2 Gew.-% 1,1-GPS, 37 Gew.-% 1,6-GPS und 53 Gew.-% 1,1-GPM, bezogen auf das Gewicht des Süßungsmittelgemisches, | 19,54 kg |
| Acesulfam-K | 30 g |
| Aspartam | 30 g |
| Pfefferminzaroma | 200 g |
| Menthol | 100 g |
| Magnesiumstearat | 100 g |

### Herstellung

Die Komponenten werden gemischt und in einer Rundläuferpresse Fette P 1200 unter folgenden Bedingungen gepreßt:
Preßkraft 20 bis 70 kN

Zur Herstellung von Lutschtabletten wird vorzugsweise ein Gemisch mit einem höheren 1,6-GPS-Gehalt und für die Herstellung von Kautabletten ein Gemisch mit einem höheren 1,1-GPM-Gehalt verwendet. In beiden Fällen sind keine Hilfsmittel notwendig.

Man erhält homogen verpreßte, harte und gut lösliche Komprimate.

### Beispiel 2: Auflösekinetik von Komprimaten

Zum Vergleich des Löslichkeitsverhaltens von Komprimaten, die erfindungsgemäß 1,1-GPS und im Vergleich dazu Isomalt^{R} und Saccharose enthalten, wurden Auflösekinetiken der unterschiedlichen Komprimate aufgenommen. Die Isomalt^{R} enthaltenden Komprimate enthielten kein 1,1-GPS, sondern sind folgendermaßen zusammengesetzt: 19,54 kg Isomalt^{R}, 200 g Pfefferminzaroma, 100 g Menthol, 100 g Magnesiumstearat, 30 g Acesulfam-K, 30 g Aspartam.

Die Saccharose enthaltenden Komprimate enthielten ebenfalls kein 1,1-GPS, sondern waren folgendermaßen zusammengesetzt: 19,6 kg Saccharose, 200 g Eucalyptus-Menthol, 100 g Menthol, 100 g MagnesiumStearat.

Die erfindungsgemäßen 1,1-GPS enthaltenden Komprimate wurden gemäß Beispiel 1 hergestellt.

Das Auflöseverhalten wurde in einer Lösung gemäß LMBG § 35 (Lebensmittel- und Bedarfsmittelgesetz) bei 37° C bestimmt. Die Menge an eingesetztem Lösungsmittel und Komprimaten wurde so gewählt, daß bei vollständiger Auflösung der Komprimate eine 10%ige Lösung gebildet wird. In Abhängigkeit von der Zeit wurde die Dichtezunahme in der Lösung ermittelt und daraus die Konzentration in g Trockensubstanz pro 100 g Lösung (c, siehe Figur) bestimmt.

Die Figur zeigt, daß die 1,1-GPS enthaltenden Komprimate eine erhöhte Löslichkeit gegenüber Komprimaten aufweisen, die Isomalt^{R} enthalten. Auch gegenüber Zucker-haltigen Komprimaten ergibt sich eine veränderte Auflösekinetik, das heißt, 1,1-GPShaltige Komprimate gehen, insbesondere zu Beginn des Auflösungsvorgangs, schneller in Lösung. Die erfindungsgemäßen Komprimate erweitern daher in vorteilhafter Weise das Spektrum an zur Verfügung stehenden Trägern, beispielsweise für die Arzneimittelapplikation.

### Beispiel 3: Komprimierversuche

Um die erfindungsgemäßen Komprimate im Vergleich zu Komprimaten aus Isomalt^{R} und Saccharose im Hinblick auf den zu ihrer Herstellung notwendigen Preßdruck und der erzielten Härte zu vergleichen, wurden die folgenden Komprimierversuche durchgeführt:

Die Zusammensetzung der erfindungsgemäßen Komprimate entsprach der Rezeptur des Beispiels 1.

Als Vergleichskomprimate wurden ein Isomalt^{R} und ein Saccharose-Gemisch der in Beispiel 2 beschriebenen Zusammensetzung verwendet.

Die Komprimierversuche wurden mit einer Rundläuferpresse Fette P 1200 durchgeführt, wobei der Stempel rund und facettiert war. Der Durchmesser des Stempels betrug 20 mm. Die Rundläuferpresse wurde mit Rundstabrädern ausgerüstet.

Zur Verpressung von Isomalt^{R} wurde ein Vorpreßdruck von 24,3 kN und ein Hauptpreßdruck von 65,4 kN benötigt, wobei ein Komprimat mit der Härte 76 N erhalten wurde. Zur Verpressung von Saccharose wurde ein Vorpreßdruckvon 24,0 kN und ein Hauptpreßdruck von 65,0 kN benötigt, wobei ein Komprimat mit der Härte 128 N erhalten wurde. Im Gegensatz dazu wurde für die Verpressung der erfindungsgemäßen Komprimate ein Vorpreßdruck von 28,3 kN und ein Hauptpreßdruck von 49,4 kN benötigt, wobei die erhaltenen Komprimate eine Härte von 204 N aufwiesen. Die Herstellung der erfindungsgemäßen Komprimate kann also mit einem geringeren Hauptpreßdruck geschehen, wobei in vorteilhafter Weise härtere Komprimate als im Stand der Technik resultieren.

## Patentansprüche

1. Komprimat, enthaltend 50 bis 99 Gew.-%, bezogen auf das Gewicht des Komprimates, eines Süßungsmittelgemisches aus 1,6-GPS (6-O-α-D-Glucopyranosyl-D-sorbit), 1,1-GPS (1-O-α-D-Glucopyranosyl-D-sorbit) und 1,1-GPM (1-O-α-D-Glucopyranosyl-D-mannit), wobei das Süßungsmittelgemisch 10 bis 50 Gew.-% 1,6-GPS, 2 bis 20 Gew.-% 1,1-GPS und 30 bis 70 Gew.-% 1,1-GPM, bezogen auf das Gewicht des Süßungsmittelgemisches, enthält.

2. Komprimat nach Anspruch 1, wobei das Komprimat zusätzlich Monosaccharide, Disaccharide, Monosaccharidalkohole, Disaccharidalkohole, Stärke, Stärkederivate, Cellulose, Cellulosederivate oder Inulin enthält.

3. Komprimat nach einem der Ansprüche 1 oder 2, wobei das Komprimat zusätzlich einen Intensiv-Süßstoff, insbesondere Acesulfam K, Thaumatin, Glycyrrhizin, Saccharin oder Cyclamat enthält.

4. Komprimat nach einem der Ansprüche 1 bis 3, wobei das Komprimat zusätzlich Aromastoffe, insbesondere Frucht- oder Pfefferminzaroma, Farbstoffe oder Sprengmittel, wie Bicarbonat oder Carboxylmethylcellulose enthält.

5. Komprimat nach einem der Ansprüche 1 bis 4, wobei das Komprimat zusätzlich einen pharmazeutisch aktiven Wirkstoff, insbesondere ein Enzym, ein Coenzym, ein Antibiotikum, einen mikrobizid oder fungizid wirkenden Stoff, Nicotin, Coffein, Menthol oder Eukalyptus enthält.

6. Komprimat nach einem der Ansprüche 1 bis 5, wobei das Komprimat in Form einer Lutsch- oder Kautablette vorliegt.

## Claims

1. Tablet, containing 50 to 99% by weight relative to the weight of the tablet, of a sweetening mixture made of 1,6-GPS (6-O-α-D-glucopyranosyl-D-sorbitol), 1,1-GPS (1-O-α-D-glucopyranosyl-D-sorbitol) and 1,1-GPM (1-O-α-D-glucopyranosyl-D-mannitol), the sweetening mixture containing 10 to 50% by weight 1,6-GPS, 2 to 20% by weight 1,1-GPS and 30 to 70% by weight 1,1-GPM relative to the weight of the sweetening mixture.

2. Tablet according to claim 1, the tablet additionally containing monosaccharides, disaccharides, monosaccharide alcohols, disaccharide alcohols, starch, starch derivatives, cellulose, cellulose derivatives or inulin.

3. Tablet according to one of the claims 1 or 2, the tablet additionally containing an intensive sweetener, in particular acesulfame K, thaumatin, glycyrrhizine, saccharin or cyclamate.

4. Tablet according to one of the claims 1 to 3, the tablet additionally containing flavourings, in particular fruit or peppermint flavouring, colourings or dissolution agents such as bicarbonate or carboxylmethylcellulose.

5. Tablet according to one of the claims 1 to 4, the tablet additionally containing a pharmaceutically active agent, in particular an enzyme, a coenzyme, an antibiotic, a microbicidally or fungicidally effective agent, nicotine, caffeine, menthol or eucalyptus.

6. Tablet according to one of the claims 1 to 5, the tablet being in the form of a tablet for sucking or chewing.

## Revendications

1. Comprimé, contenant de 50 à 99 % en poids, rapporté au poids du comprimé, d'un mélange édulcorant à base de 1,6-GPS(6-O-α-D-Glucopyranosyl-D-sorbitol), 1,1-GPS (1-O-α-D-Glucopyranosyl-D-sorbitol) et de 1,1-GPM (1-O-α-D-Glucopyranosyl-D-mannitol), dans lequel le mélange édulcorant contient de 10 à 50 % en poids de 1,6-GPS, de 2 à 20 % en poids de 1,1-GPS et de 30 à 70 % en poids de 1,1-GPM rapporté au poids du mélange édulcorant.

2. Comprimé selon la revendication 1, dans lequel le comprimé contient en supplément des monosaccharides, des disaccharides, des alcools de monosaccharides, des alcools de disaccharides, des amidons, des dérivés d'amidon, de la cellulose, des dérivés de cellulose ou de l'inuline.

3. Comprimé selon l'une quelconque des revendications 1 ou 2, dans lequel le comprimé contient en supplément un édulcorant intense, en particulier l'acésulfame K, la thaumatine, la glycirrhizine, la saccharine ou le cyclamate.

4. Comprimé selon l'une quelconque des revendications 1 à 3, dans lequel le comprimé contient en supplément des substances aromatiques en particulier des arômes de fruit ou de menthe poivrée, des colorants ou des agents d'éclatement comme un bicarbonate ou la carboxylméthylcellulose.

5. Comprimé selon l'une quelconque des revendications 1 à 4, dans lequel le comprimé contient en supplément un principe pharmaceutiquement actif, en particulier un enzyme, un coenzyme, un antibiotique, une substance à action microbicide ou fongicide, la nicotine, la caféine, le menthol ou l'eucalyptus.

6. Comprimé selon l'une quelconque des revendications 1 à 5, dans lequel le comprimé se présente sous forme d'un comprimé à sucer ou à mâcher.
